# EUROPEAN PATENT SPECIFICATION

(11) **EP 4 316 367 B1**
(45) Date of publication and mention of the grant of the patent: **24.09.2025**
(21) Application number: 23189228.2
(22) Date of filing: 02.08.2023
(51) Int. Cl.: A61B 5/11, A61B 5/00

(54) **WEARABLE DEVICE, MOVEMENT INFORMATION ACQUISITION CONTROL METHOD AND PROGRAM**
WEARABLE-VORRICHTUNG, BEWEGUNGSINFORMATIONSERFASSUNGSSTEUERUNGSVERFAHREN UND PROGRAMM
DISPOSITIF PORTABLE, PROCÉDÉ ET PROGRAMME DE COMMANDE D'ACQUISITION D'INFORMATIONS DE MOUVEMENT

(30) Priority: 04.08.2022 JP 2022124520
(43) Date of publication of application: 07.02.2024
(73) Proprietor: Casio Computer Co., Ltd., Tokyo 151-8543 (JP)
(72) Inventor: OSHITA, Yuuki, Tokyo, 205-8555 (JP)
(74) Representative: Plougmann Vingtoft a/s

(56) References cited:
- US-A1- 2015 172 854
- US-A1- 2017 027 523
- US-A1- 2018 364 064
- US-A1- 2020 330 003

## Description

### BACKGROUND OF THE INVENTION

### Technical Field

The present disclosure relates to a wearable device, a movement information acquisition control method, and a program therefor.

### Background Art

Conventionally, there is a wearable device that is fixed to a user's arm (including wrist), leg, torso, or the like and measures the user's activity and vitals. This wearable device receives radio waves from positioning satellites associated with the GNSS (Global Navigation Satellite System) and performs positioning (satellite positioning) to identify the position of the user who moves along with the activity so as to acquire movement associated information such as movement histories and moving distances, etc.

Satellite positioning is difficult underground, in tunnels, and indoors, where radio waves cannot be received from positioning satellites. It is also difficult to obtain accurate positioning results in places where radio waves are difficult to receive from wide directions, such as valleys between skyscrapers and mountains. In addition, there are cases where a portable device performs positioning operations intermittently in order to reduce power consumption. In these cases, the user's relative walking movement amount is calculated using a physical quantity measurement sensor such as an acceleration sensor or an orientation sensor, and the measured results are added to the reference position to determine the movement history between the positions determined by satellite positioning. Japanese Patent Application Laid-Open No. 2012-98137, which has technology related to dead reckoning, discloses that data related to relative movement can be obtained more accurately by changing the detection pattern for acceleration according to the holding state of the aircraft during measurement with dead reckoning.

US 2015/172854 A1 discloses a method includes a step for receiving session data from at least one mobile device, wherein each instance of the received session data includes one or more global positioning system (GPS) data points, one or more sensor data points, and one or more WiFi scan data points, wherein the one or more WiFi scan data points are associated with one or more wireless access points (WAPs). The method also includes a step for calculating a location of at least one of the one or more WAPs using at least a portion from each of the received one or more global positioning system (GPS) data points, the one or more sensor data points, and the one or more WiFi scan data points.

US 2017/027523 A1 discloses a biometric monitoring device is used to determine a user's heart rate by using a heartbeat waveform sensor and a motion detecting sensor. **In** some embodiments, the device collects output data from the heartbeat waveform sensor and output data from the motion detecting sensor, determines an expected heart rate range from the output data from the motion detecting sensor, and determines the user's heart rate within the expected heart rate range using the output data from the heartbeat waveform sensor.

US 2018/364064 A1 discloses an electronic device, comprising: a processor; and and a first storage unit that stores programs to be executed by the processor, wherein the processor reads out the programs stored on the first storage unit so as to perform the following when a user moves: acquiring positional information of a first location of the user that is determined using radio waves from navigation satellites; acquiring positional information of a second location to which the user traveled from the first location, the positional information of the second location being determined using radio waves from navigation satellites; performing a first distance obtaining process of obtaining, without using map information, a first distance between the first location and the second location based on the acquired positional information of the first location and the acquired positional information of the second location; performing a second distance obtaining process of obtaining, without using map information, a second distance between the first location and the second location, by using dead reckoning based on a step count and a step length of the user as the user traveled from the first location to the second location; and performing a step length correction process of correcting the step length of the user that is used in the dead reckoning on the basis of the first distance obtained by the first distance obtaining process and the second distance obtained by the second distance obtaining process.

US 2020/330003 A1 dicloses an apparatus (100) for passive scanning of an object. The apparatus comprises a distance sensing unit (110) adapted to measure distances to a plurality of points of the object, an orientation sensing unit (120) adapted to determine orientations of the distance sensing unit (110), and a processor (140) adapted to derive information about the object or a scene in which the object is used based on the measured distances and orientations of the distance sensing unit (110).

### SUMMARY OF THE INVENTION

Features and advantages of the disclosure will be set forth in the descriptions that follow and in part will be apparent from the description, or may be learned by practice of the disclosure. The objectives and other advantages of the disclosure will be realized and attained by the structure particularly pointed out in the written description and claims thereof as well as the appended drawings.

According to a first aspect of the present invention there is provided a wearable device, comprising:
a positioning processing unit that performs positioning of the wearable device based on radio waves received from satellites;
a measurement unit that performs measurements relating to an orientation and movement of the wearable device;
an acquisition unit that acquires information representing a user's exercise type; and
a control unit, wherein the control unit determines whether to calculate a movement amount of the wearable device per set intervals based on measurement results of the measurement unit during a period in which the positioning based on radio waves received from satellites is not obtained, in accordance with the user's exercise type acquired by the acquisition unit,
wherein when the control unit determines that the positioning based on radio waves received from satellites is intermittently performed with an idle period in between and the movement amount is not to be calculated based on measurement results of the measurement unit, the control unit performs additional positioning based on radio waves received from satellites if a change in traveling direction is detected by the measurement unit during the idle period.

According to a further aspect of the present invention there is provided a method for controlling acquisition of movement information of a wearable device, performed by the wearable device, the wearable device including a positioning processing unit that performs positioning of the wearable device based on radio waves received from satellites and a measurement unit that performs measurements relating to an orientation and movement of the wearable device, the method comprising:
acquiring information representing a user's exercise type; and
determining whether to calculate a movement amount of the wearable device per set intervals based on measurement results of the measurement unit during a period in which the positioning based on radio waves received from satellites is not obtained, in accordance with the user's exercise type,
wherein when determining that the positioning based on radio waves received from satellites is intermittently performed with an idle period in between and the movement amount is not to be calculated based on measurement results of the measurement unit, performing additional positioning based on radio waves received from satellites if a change in traveling direction is detected by the measurement unit during the idle period.

According to a further aspect of the present invention there is provided a program executable by a computer in a wearable device that includes a positioning processing unit that performs positioning of the wearable device based on radio waves received from satellites and a measurement unit that performs measurements relating to an orientation and movement of the wearable device, the non-transitory storage medium storing a program executable by the computer, the program causing the computer to function as:
an acquisition unit that acquires information representing a user's exercise type; and
a calculation determination unit that determines whether to calculate a movement amount of the wearable device per set intervals based on measurement results of the physical sensor during a period in which the positioning based on radio waves received from satellites is not obtained, in accordance with the user's exercise type,
wherein when determining that the positioning based on radio waves received from satellites is intermittently performed with an idle period in between and the movement amount is not to be calculated based on measurement results of the measurement unit,
performing additional positioning based on radio waves received from satellites if a change in traveling direction is detected by the measurement unit during the idle period.

It is to be understood that both the foregoing general description and the following detailed description are exemplary and explanatory, and are intended to provide further explanation of the disclosure as claimed.

### BRIEF DESCRIPTION OF THE DRAWINGS

FIG. 1 is a block diagram showing the functional configuration of an electronic timepiece.
FIGs. 2A-2B are diagrams showing examples of position acquisition when movement histories are acquired for a run-walk activity and a non-run-walk activity, respectively.
FIG. 3 is a flowchart showing a control procedure of movement history acquisition processing.
FIGs. 4A-4B are diagrams showing examples of moving distance acquisition when moving distances are acquired for a run-walk activity and a non-run-walk activity, respectively.
FIG. 5 is a flowchart showing a control procedure of movement amount measurement control processing.

### DETAILED DESCRIPTION OF EMBODIMENTS

In dead reckoning, when the movement is not on foot, it has been difficult to appropriately determine the operational interval for the user's movement and the movement speed of the user's movement per operational interval, and as a result, the deviation from the actual movement history tends to increase, which is a problem.

An object of the present disclosure is to provide a wearable device, movement information acquisition control method, and a storage medium that stores a computer executable program that perform such a method that reflect a user's exercise type more and that can switch modes more appropriately.

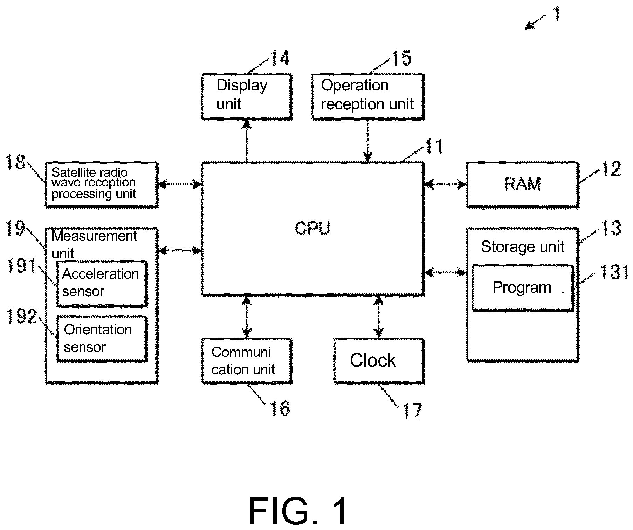

Embodiments will be described below with reference to the drawings. FIG. 1 is a block diagram showing the functional configuration of an electronic timepiece 1, which is a wearable device according to this embodiment. The electronic timepiece 1 includes a CPU 11 (Central Processing Unit) (control unit), a RAM 12 (Random Access Memory), a storage unit 13, a display unit 14, an operation reception unit 15 (acquisition unit), a communication unit 16, a clock unit 17, a satellite radio wave reception processing unit 18 (positioning processing unit), and a measurement unit 19, etc.

The CPU 11 is a processor that performs arithmetic processing and controls the overall operation of the electronic timepiece 1. A single processor may be used, or a plurality of processors may operate in parallel or independently depending on the application. Further, the CPU 11 may be a dedicated microcomputer or the like in addition to or instead of the general-purpose CPU. Generally, in this disclosure, the word "processor" can be a single processor or a plurality of separate processors that operate in parallel or independently.

The RAM 12 is, for example, a DRAM, and provides a work memory space to the CPU 11 to store temporary data.

At least the CPU 11 and RAM 12 are included in the computer of the wearable device of this embodiment.

The storage unit 13 is a non-volatile memory such as a flash memory. The storage unit 13 stores a program 131, setting data, and the like.

The display unit 14 displays at least the time based on the control of the CPU 11. The display unit 14 may have a digital display screen such as a liquid crystal display, or may have a plurality of hands (hour hand, minute hand, etc.), a gear train (wheel train mechanism) and a stepping motor or the like for rotating the wheel train mechanism

The operation reception unit 15 receives an input operation from the outside such as a user and outputs a reception signal to the CPU 11. The operation reception unit 15 has, for example, a push button switch, and detects a pressing operation of the push button switch to output a reception signal. Further, the operation reception unit 15 may have a crown that can be pulled out and rotated.

The communication unit 16 controls communications with external devices. The communication unit 16 has, for example, a network card for LAN communication, a communication module for short-range wireless communication using Bluetooth (registered trademark), and the like, and for example, it controls transmission of activity measurement results of the electronic timepiece 1 to an external device.

The clock unit 17 counts clock signals with a certain oscillation frequency generated by an oscillation circuit (not shown) and outputs the current date and time. The clock unit 17 may be one in which the CPU 11 counts the date and time using software.

The satellite radio wave reception processing unit 18 has an antenna (not shown), receives radio waves from a plurality (at least four) of positioning satellites in the sky, and performs positioning operations in which the current date and time and the current position of the electronic timepiece 1 are calculated based on the received contents and the timing difference thereof. The positioning satellites from which radio waves are received include those related to GNSS (Global Positioning System) such as Global Positioning System (GPS), Quasi-Zenith Satellite System (QZSS), GLONASS, and Galileo. The satellite radio wave reception processing unit 18 has a reception processing unit that receives radio waves from positioning satellites and demodulates and decodes the signals, and an arithmetic processing unit that performs positioning calculations based on the contents of the signals. The reception processing unit and/or the arithmetic processing unit may be configured by a combination of a CPU (not shown) that is different from the CPU 11 and a circuit for demodulation and decoding. Here, the arithmetic processing unit may be realized as a function of the CPU 11.

The measurement unit 19 has physical sensors that measure the orientation and movement of the timepiece itself, and outputs the measurement results of each sensor to the CPU 11. The physical sensors include, for example, an acceleration sensor 191 and an orientation sensor 192.

The acceleration sensor 191 measures acceleration in three axial directions. Although the three axial directions may be determined appropriately (for example, two axes parallel to the display surface of the display unit 14 and one axis perpendicular to the display surface), since the gravitational acceleration can be measured, it is also possible to separate components in directions in the horizontal plane and in the gravitation direction by coordinate transformation. The orientation sensor 192 is a sensor that measures the earth's magnetic field, and can obtain the orientation of magnetic north by measuring the magnetic field strength in three axial directions.

The electronic timepiece 1 of this embodiment is, for example, a wristwatch-type electronic device that can be worn on the wrist with a band.

Next, the position information acquisition operation in the electronic timepiece 1 will be described.

Once the electronic timepiece 1 of the present embodiment accepts (acquires) the setting as to which activity (exercise type) is to be performed by the user by the user's input operation performed on the operation reception unit 15, it starts measuring for the activity. Activity measurement ends when an instruction to end activity measurements is received by an input operation, when a specified maximum duration elapses, or when a reference time elapses without movement being measured.

The activities mentioned here are those involving movement of position in the horizontal directions, and include the run-walk group activities (movement due to running or walking), such as walking (walking, strolling, hiking, trekking, mountain climbing) and running (running, jogging, cross-country, trail running), etc., as well as the non-run-walk group activities (not involving the user's running or walking), such as bicycling (cycling, long ride), skiing (downhill), snowboarding, swimming, etc. Note that cross-country skiing may be regarded as a run-walk activity.

With the electronic watch 1, for run-walk group activities, satellite positioning operations are performed intermittently, and the position between them is complemented by adding the amount of movement by dead reckoning. In dead reckoning, the user's relative run-walk movement amount is calculated using the measurement unit 19 such as the acceleration sensor 191 and the orientation sensor 192. That is, Pedestrian Dead-Reckoning (PDR) is used to supplement the movement trajectory. On the other hand, as described above, since the technology of dead reckoning cannot obtain sufficient accuracy except for run-walk type movements, for non-run-walk group activities, the amount of movement is not calculated by dead reckoning. In this case, based on the measurement results of the measurement unit 19, such as the acceleration sensor 191 and the orientation sensor 192, which respectively measure the movement and orientation of the device itself, it is determined whether the direction of movement of the user has changed. And if the movement direction is found to have changed, the device performs satellite positioning operation. That is, the electronic timepiece 1 determines whether or not to calculate the moving distance and the current position by dead reckoning according to the activity (exercise type).

Here, since the measurements by the measurement unit 19 for dead reckoning during the run-walk activity consumes sufficiently less power than the operation of the satellite radio wave reception processing unit 18, it is not necessary to stop the operation of measurements by the measurement unit 19 even when positioning is being performed by the satellite radio wave reception processing unit 18, and the operation may be continued during the satellite positioning operation period. However, in order to operate the electronic timepiece 1 with lower power consumption, the measurement unit 19 for dead reckoning may be operated only when the satellite radio wave reception processing unit 18 is idle. During a non-run-walk activity, only the orientation sensor 192 may operate as necessary in the manner as described above. Or alternatively, the operation of the measurement unit 19 may be continued regardless of the activities considering other functions it may be assigned to perform.

The directional change in this case may be detected by appropriately considering cumulative directional changes and average directional changes so that the detection of at least gradual changes in direction, such as curves, is possible while avoiding the detection of each minute turn, etc., during skiing. The criteria for the consideration and the reference value (lower limit) of the angle change to be detected may be determined according to the type of activity.

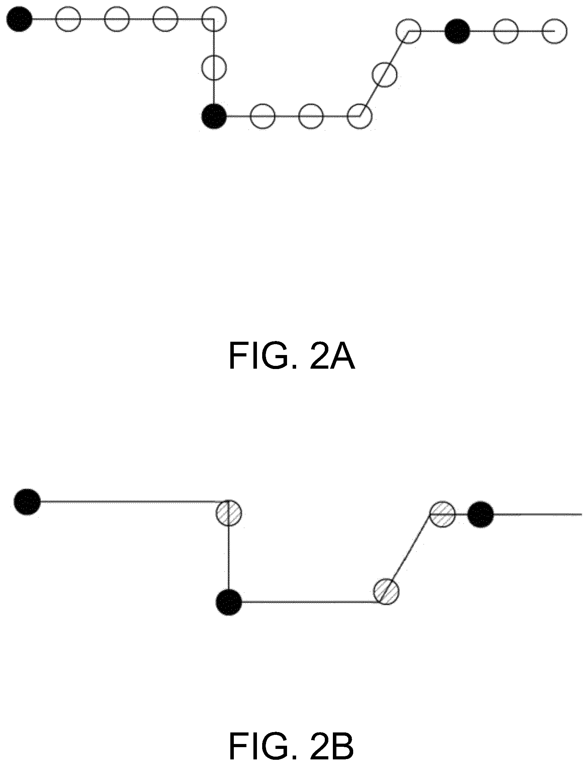

FIGs. 2A-2B are diagrams showing an example of position acquisition when movement histories are acquired for a run-walk activity and a non-run-walk activity, respectively.

As shown in FIG. 2A, in the run-walk activity, for example, intermittent satellite positioning is performed at fixed intervals (black circles) with rest/idle periods (periods in which positioning is not performed) in between. The current position is calculated by calculating the direction of movement and the distance traveled (movement amount) for each time using dead reckoning periodically at time intervals (the white circles) shorter than the rest/idle period, and adding it to the reference satellite positioning position. At this time, as the number of times of addition increases, the position obtained by the dead reckoning also accumulates errors, so it may be corrected by a known technique based on the most recent satellite positioning results.

On the other hand, as shown in FIG. 2B, satellite positioning is intermittently (at a regular interval) performed even in the non-run-walk activity (black circles), but the current position is not calculated by dead reckoning during the set interval between the satellite positionings. Instead, when a change in the direction of travel is detected by the measurement of the measurement unit 19 (especially the orientation sensor 192), satellite positioning is additionally performed by the satellite radio wave reception processing unit 18 to determine the position at the vicinity of the change point of the direction of travel (hatched circles). In this way, even in a non-run-walk activity, measurement is performed that enables an outline of the movement route to be obtained without significantly increasing power consumption.

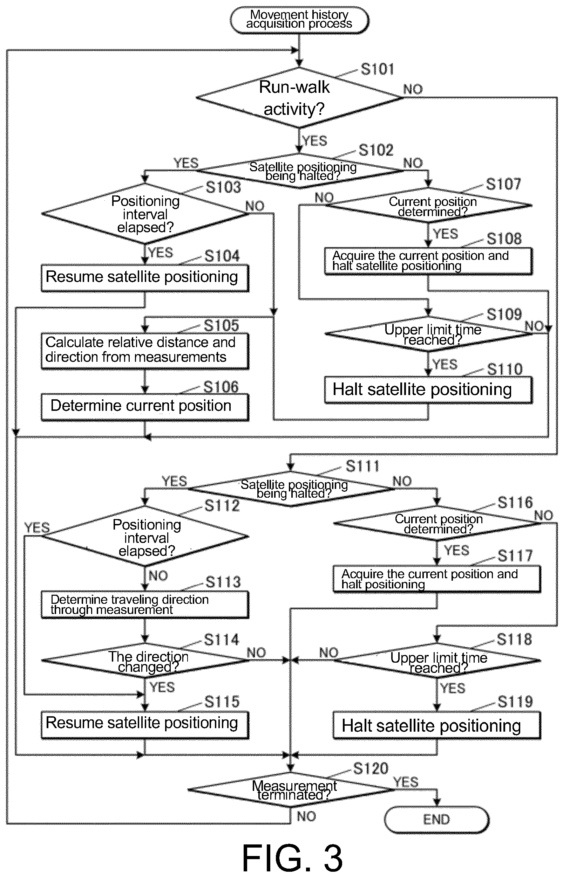

FIG. 3 is a flowchart showing the control procedure by the CPU 11 of the movement history acquisition process executed by the electronic timepiece 1. This movement history acquisition process is started when the operation reception unit 15 acquires instructions for selecting an activity and starting movement history measurement.

The CPU 11 determines whether the selected activity is of run-walk type or not (step S101). When it is determined that the system is a run-walk group activity ("YES" in step S101), the CPU 11 determines whether or not satellite positioning is being halted or not (step S102).

When it is determined that satellite positioning is being halted ("YES" in step S102), the CPU 11 determines whether or not the positioning interval has elapsed since the previous satellite positioning (step S103). When it is determined that the positioning interval has elapsed ("YES" in step S103), the CPU 11 causes the satellite radio wave reception processing unit 18 to resume satellite positioning (step S104). Then, the processing of the CPU 11 proceeds to step S120.

When it is determined that the positioning interval has not elapsed ("NO" in step S103), the CPU 11 calculates the relative movement distance and the movement direction from the measurement values of the measurement unit 19 (step S105). ). The CPU 11 then determines the current position by adding the relative movement distance and movement direction to the previous position (step S106). Then, the processing of the CPU 11 proceeds to step S120.

When it is determined in the determination process of step S102 that the satellite positioning is not being halted ("NO" in step S102), the CPU 11 determines whether or not the current position is determined by satellite positioning (step S107). ). If it is determined that the current position has been determined ("YES" in step S107), the CPU 11 acquires the specified current position and halts satellite positioning (step S108). Then, the processing of the CPU 11 proceeds to step S120.

When it is determined that the current position is not determined by satellite positioning ("NO" in step S107), the CPU 11 determines whether or not the upper limit time has elapsed since the start (restart) of the processing for positioning (step S109). If it is determined that the upper limit time has not been reached ("NO" in step S109), the process of the CPU 11 returns to step S101. If it is determined that the upper limit time has elapsed ("YES" in step S109), the CPU 11 halts the satellite positioning operation by the satellite radio wave reception processing unit 18 (step S110), and then proceeds to step S105.

When it is determined in the determination process of step S101 that the selected activity is not a run-walk group activity ("NO" in step S101), the CPU 11 determines whether or not satellite positioning is being halted. (Step S111).

When it is determined that satellite positioning is being halted ("YES" in step S111), the CPU 11 determines whether or not a positioning interval has elapsed since the previous satellite positioning (step S112). When it is determined that the positioning interval has elapsed ("YES" in step S112), the CPU 11 resumes satellite positioning by the satellite radio wave reception processing unit 18 (step S115). Then, the processing of the CPU 11 proceeds to step S120.

When it is determined that the positioning interval has not elapsed ("NO" in step S112), the CPU 11 determines the traveling direction from the measurement value of the measuring unit 19 (step S113). The CPU 11 determines whether or not a change in traveling direction has been detected (step S114). If it is determined that a change in traveling direction has been detected ("YES" in step S114), the processing of the CPU 11 proceeds to step S115. If it is determined that no change in traveling direction has been detected ("NO" in step S114), the processing of the CPU 11 proceeds to step S120.

When it is determined in the determination process of step S111 that the satellite positioning is not being halted ("NO" in step S111), the CPU 11 determines whether or not the current position is determined by satellite positioning (step S116). ). If it is determined that the current position has been determined ("YES" in step S116), the CPU 11 acquires the specified current position and halts satellite positioning by the satellite radio wave reception processing unit 18 (step S117). Then, the processing of the CPU 11 proceeds to step S120.

When it is determined that the current position has not been determined ("NO" in step S116), the CPU 11 determines whether or not the upper time limit of continuing satellite positioning operation has elapsed (step S118). If it is determined that the upper limit time has not been reached ("NO" in step S118), the process of the CPU 11 proceeds to step S120. If it is determined that the upper limit time has elapsed ("YES" in step S118), the CPU 11 halts the satellite positioning operation by the satellite radio wave reception processing unit 18 (step S119). Then, the processing of the CPU 11 proceeds to step S120.

When proceeding to the process of step S120, the CPU 11 determines whether or not an instruction to terminate activity measurement has been acquired (step S120). If it is determined that the instruction to terminate the measurement has not been acquired ("NO" in step S120), the process of the CPU 11 returns to step S101. When it is determined that the instruction to terminate the measurement has been acquired ("YES" in step S120), the CPU 11 ends the process for the acquisition of the movement history, and termiantes the movement history acquisition process.

The processes of steps S101, S102, and S111 constitute a calculation determination unit of this embodiment.

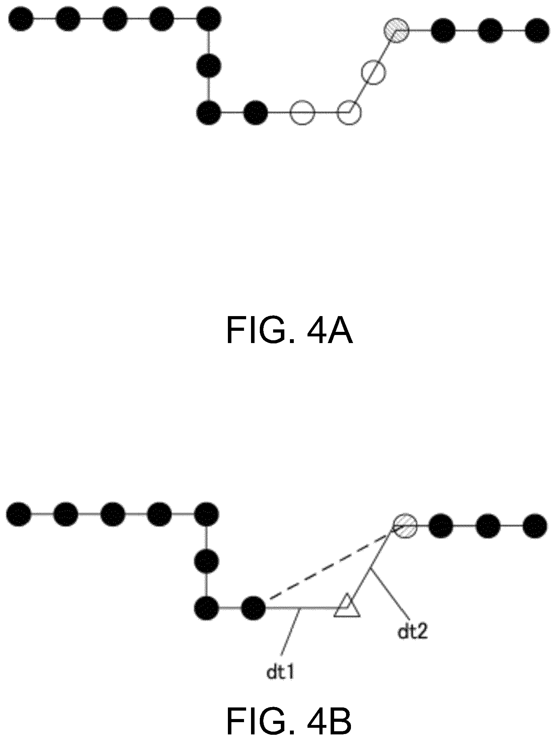

FIGs. 4A-4B are diagrams showing examples of moving distance acquisition when moving distances are acquired for a run-walk activity and a non-run-walk activity, respectively.

As shown in FIG. 4A, if, during the process of obtaining the movement distance from the amount of change in the current positions (black circles) obtained by performing satellite positioning in a run-walk activity, the satellite positioning result could not be obtained (i.e., if the acquisition fails, for example, in a tunnel, underground, in a building or in a deep valley of a mountain, as described above), the process is switched to relative movement distance measurement by dead reckoning, and the total movement distance can be obtained by accumulating the relative movement distances (in this case, the current positions indicated by the white circles do not necessarily have to be specified).

When the acquisition of satellite positioning results is resumed, the distance moved from the previous position cannot be obtained from the first position after the restart (the circle hatched with diagonal lines), so the distance moved from the previous position to this timing can be the distance obtained by dead reckoning. When the current position is determined by accumulating the relative movement distances by dead reckoning, the distance connecting the positions accumulate measurement errors and the current position obtained by satellite positioning may deviate greatly from the accurate value.

On the other hand, in FIG. 4B, if, during the process of obtaining the movement distance from the amount of change in the current positions (black circles) obtained by performing satellite positioning in a non-run-walk activity, the satellite positioning result could not be obtained, dead reckoning is not performed, and as soon as the next satellite positioning results become available, the distance between the current position (hatched circle) and the previous position (the distance between the two positions adjacent in time that are determined by satellite positioning) is obtained as the moving distance (dashed line).

Alternatively, similarly to the above, based on the measurement result of the orientation sensor 192 of the measurement unit 19, the timing and the direction of travel whenever a change in moving direction occurred may be determined, and the moving distances to respective directional movements are distributed between the two points where the positionings were successful in accordance with the respective time intervals (dt1+dt2) spent in the respective directions so as to obtain the movement distance between the two points that are determined by satellite positioning. For example, as shown in FIG. 4B, if there is only one bend in moving direction, the intersection (as indicated by the triangle in FIG. 4B) of a straight line extending from the position immediately before the positioning failure in the travel direction at that time and another straight line on the horizontal plane where the distances from the two points before and after the positioning failure to the intersection are distributed by the ratio of dt1:dt2 can be regarded as a position at which the moving direction has changed in determining the movement distance. Alternatively, the moving distance may be calculated by multiplying the average value of the moving speeds before and after the positioning failure period by the duration of the positioning failure. In this case, it is unnecessary to detect a change in the direction of travel by the measurement unit 19. Furthermore, in this case, depending on the type of activity, the upper limit of the moving distance within the positioning failure period may be set for the linear distances between the two positions, so that temporary movement stop during the positioning failure period can be considered.

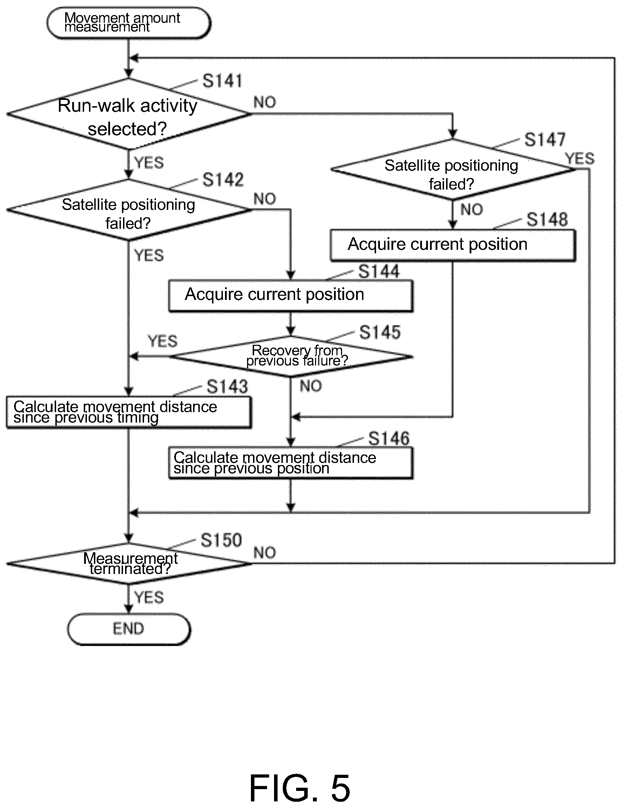

FIG. 5 is a flow chart showing a control procedure by the CPU 11 for movement amount measurement control processing.

This process is started when an instruction for selecting an activity and starting movement amount measurement is acquired by the operation reception unit 15 or the like.

The CPU 11 determines whether or not a run-walk group activity has been selected (step S141). When it is determined that a run-walk group activity is selected ("YES" in step S141), the CPU 11 determines whether acquisition of the satellite positioning result (current position) has failed (step S142).

If it is determined that acquisition of the result of satellite positioning has failed ("YES" in step S142), the CPU 11 calculates the movement distance since the previous timing at which the movement distance was obtained by the pedestrian dead reckoning (PDR), based on the measurement result of the measurement unit 19 (step S143). Then, the processing of the CPU 11 proceeds to step S150.

When it is determined that acquisition of satellite positioning results has not failed ("NO" in step S142), the CPU 11 acquires the current position (step S144). The CPU 11 then determines whether or not that was a recovery from the previous failure in acquisition of the positioning result (step S145).

If it is determined that it is not a recovery from the previous failure ("NO" in step S145), the CPU 11 obtains the difference between the previous position and the current position so as to calculate the movement amount from the position obtained by the previous satellite positioning (step S146). Then, the processing of the CPU 11 proceeds to step S150. If it is determined to be a recovery from the previous failure ("YES" in step S145), the processing of the CPU 11 proceeds to step S143.

When it is determined in the determination process of step S141 that a run-walk activity is not selected (i.e., a non-run-walk activity is selected) ("NO" in step S141), the CPU 11 determines whether or not satellite positioning result acquisition has failed (step S147). If it is determined that acquisition of the result of satellite positioning has not failed ("NO" in step S147), the CPU 11 acquires the current position (step S148). Then, the processing of the CPU 11 proceeds to step S146. In this case, when a recovery from the positioning failure occurs, the "previous position" in step S146 is the position obtained by positioning before the positioning failure occurs.

When it is determined that acquisition of the satellite positioning result has failed ("YES" in step S147), the processing of the CPU 11 proceeds to step S150.

When proceeding to the process of step S150, the CPU 11 determines whether or not an activity measurement termination instruction has been acquired (step S150). If it is determined that the termination instruction has not been acquired ("NO" in step S150), the process of the CPU 11 returns to step S141. If it is determined that the termination instruction has been acquired ("YES" in step S150), all the processing for the measurement of the movement distance is terminated, and the movement amount measurement control processing is terminated.
The processes of steps S141, S142, and S147 constitutes a calculation determination unit of this embodiment.

In the above description, examples in which acquisition of the change history of the current position by intermittent reception of satellite radio waves and acquisition of the amount of movement by acquiring positioning results at short time intervals have been described. Instead, change history of the current positions may be acquired, the amount of movement may be obtained by intermittently receiving satellite radio waves and using dead reckoning in combination. In the latter case, positioning may be performed by detecting a change in traveling direction as described above.

As described above, the electronic timepiece 1, which is a wearable device according to the present embodiment, includes the satellite radio wave reception processing unit 18 that receives radio waves from positioning satellites to perform positioning; the measurement unit 19 that measures the orientation and movement of the device itself; the operation reception unit 15 as an acquisition unit that acquires information representing the type of activity (exercise) of the user; and the CPU 11. The CPU 11 determines whether or not to calculate the amount of movement for each time interval based on the measurement results of the measurement unit 19 during a period in which the results of positioning by the satellite radio wave reception processing unit 18 are not obtained, in accordance with the type of activity.

In this way, when it is possible to measure the position and amount of movement during various (multiple) activities, it is possible to switch operation modes by determining whether or not dead reckoning should be performed by acquiring the type of activity and by reflecting the user's exercise type. Thus, it is possible to avoid mixing unnatural results by not performing dead reckoning or acquisition of the results by dead reckoning when dead reckoning for the activity would not produce sufficiently accurate results. As a result, it is possible to suppress deterioration in the accuracy of the acquisition result.

Also, the CPU 11 does not calculate the movement amount when the type of activity is not a run-walk activity. The determination of the movement amount in dead reckoning for a run-walk activity can be relatively accurately performed, but in the case of movement that is not a run-walk activity, the accuracy of dead reckoning is poor. Therefore, by determining whether or not to calculate the movement amount by dead reckoning depending on whether the current activity is related to running or walking, it is possible to suppress the inclusion of unnaturally low-accuracy measurement data.

When the CPU 11 does not calculate the movement amount by dead reckoning, the CPU 11 may calculate the movement distance between the two points based on the positions of the two temporally adjacent points obtained by the satellite radio wave reception processing unit 18. That is, even when positioning is intermittent or includes a section in which satellite positioning has failed, the movement distance may be calculated simply using only successful positioning results. In this case, all the directional changes and the like are omitted, so that the movement distance becomes a minimum value, but it is possible to avoid erroneously inflating the movement distance this way.

Further, the CPU 11 can cause the satellite radio wave reception processing unit 18 to additionally perform satellite positioning even during the predetermined positioning halt period where not only positioning, but also dead reckoning is not performed, when a change in traveling direction is detected by the measurement unit 19 during the predetermined halt period. That is, when the traveling direction changes, the position at which the change occurs can be obtained by satellite positioning. Therefore, it is possible to calculate the amount of movement in line with actual movement while suppressing an increase in power consumption by performing minimal satellite positioning.

The electronic timepiece 1 also includes the operation reception unit 15 that receives an input operation as an acquisition unit. The CPU 11 determines whether or not to calculate the movement amount by dead reckoning according to the activity type determined by the operation received by the operation receiving unit 15. In this way, when the user inputs the activity type at the start of activity measurement, etc., the activity type can be easily and reliably identified, and whether or not to use dead reckoning is determined according to the activity type, thereby preventing inaccurate data contamination.

Also, the measurement unit 19 includes at least one of the acceleration sensor 191 and the orientation sensor 192. Thereby, the CPU 11 can easily identify the timing at which a change in motion such as a change in direction occurs during walking, for example.

Further, in the movement information acquisition control method of the present embodiment, information representing the type of activity of the user of the wearable device (electronic watch 1) is acquired, and whether or not to calculate the movement amount based on the measurement result of the measurement unit 19 during the period in which the result of positioning by the satellite radio wave reception processing unit 18 is not available is determined according to the type of activity..

In such a movement information acquisition control method, it is possible to determine whether or not to perform dead reckoning to switch operation modes by reflecting the type of user's activity. Therefore, it is possible to suppress the deterioration of the accuracy of the obtained result by not performing the processing and the result acquisition related to the dead reckoning for the activities for which the accurate result cannot be obtained by dead reckoning.

In addition, by installing and executing the program 131 for the movement information acquisition control method in the computer of the wearable device (electronic watch 1) having the satellite wave reception processing unit 18 and the measurement unit 19, calculation of the movement trajectory and movement amount can be performed with appropriate accuracy depending on the activities.

It should be noted that the present disclosure is not limited to the above embodiments, and various modifications are possible.

For example, in the above embodiment, satellite positioning is additionally performed when dead reckoning measurement is not to be performed, but the present invention is not limited to this. For example, the satellite positioning interval may instead be changed depending on whether dead reckoning measurement is being performed or not.

In addition, in the above embodiment, it is described that satellite positioning is additionally performed according to changes in the direction of travel in addition to intermittent satellite positioning that is to be performed at a predetermined interval, but the present invention is not limited to this. For example, when additional satellite positioning is performed, it may be determined that the next satellite positioning is performed after said predetermined interval has elapsed since that timing.

In addition, in the above embodiment, the user's exercise type is set by inputting to the operation accepting unit 15, but this is not the only option. The activity may be estimated by determining a characteristic acceleration change pattern based on the measurement result of the measurement unit 19, which depends on the activities. Also, the type of exercise may be specified based on setting data acquired via the communication unit 16.

In addition, in the above embodiment, the determination criterion is whether or not the identified activity is a run-walk group activity, but whether or not dead reckoning is used in combination may be determined based on whether or not the period of the movement pattern and the speed per such a period can be accurately determined.

Also, the electronic timepiece 1 of the present embodiment, which is a wearable device, does not always have to be worn on the body for use. For example, it may be attached to a handle or the like when performing an activity such as riding a bicycle.

Also, the wearable device is not limited to the electronic timepiece 1. An activity meter such as a smart watch, which is another terminal device worn on the arm, may be used, or a device fixed to the upper arm, head, torso, or leg may be used.

Also, in the above embodiment, the acceleration sensor 191 and the orientation sensor 192 are described as examples of the measurement unit 19, but the measurement unit 19 is not limited to this. Other sensors, such as gyro sensors, may also be used.

In the above description of the present disclosure, the storage unit 13 made up of a non-volatile memory such as a flash memory is used as an example of a computer-readable medium that stores the program 131 for the measurement control of the user's exercise. But the present invention is not limited to this. As other computer-readable media, it is possible to apply other non-volatile memories such as HDDs (Hard Disk Drives) and MRAMs, and portable recording media such as CD-ROMs and DVD disks. A carrier wave is also included in the present disclosure as a medium for providing program data via a communication line.

According to the present disclosure, the user's exercise type can be more accurately reflected in switching operation modes.

## Claims

1. A wearable device, comprising:
a positioning processing unit (18) that performs positioning of the wearable device based on radio waves received from satellites;
a measurement unit (19) that performs measurements relating to an orientation and movement of the wearable device;
an acquisition unit that acquires information representing a user's exercise type; and
a control unit (11),
wherein the control unit determines whether to calculate a movement amount of the wearable device per set intervals based on measurement results of the measurement unit during a period in which the positioning based on radio waves received from satellites is not obtained, in accordance with the user's exercise type acquired by the acquisition unit,
**characterized in that**
when the control unit determines that the positioning based on radio waves received from satellites is intermittently performed with an idle period in between and the movement amount is not to be calculated based on measurement results of the measurement unit, the control unit performs additional positioning based on radio waves received from satellites if a change in traveling direction is detected by the measurement unit during the idle period.

2. The wearable device according to claim 1, wherein the control unit does not calculate the movement amount based on measurement results of the measurement unit when the user's exercise type is an activity not involving the user's running or walking.

3. The wearable device according to claim 2, wherein when the movement amount is not calculated, the control unit calculates a movement distance between two points adjacent in time based on positions of the two points obtained by performing positioning based on radio waves received from satellites.

4. The wearable device according to claim 1, wherein the acquisition unit includes an operation reception unit (15) that receives an input operation specifying the user's exercise type.

5. The wearable device according to claim 1, wherein the measurement unit sensor includes at least one of an acceleration sensor and an orientation sensor.

6. A method for controlling acquisition of movement information of a wearable device, performed by the wearable device, the wearable device including a positioning processing unit (18) that performs positioning of the wearable device based on radio waves received from satellites and a measurement unit (19) that performs measurements relating to an orientation and movement of the wearable device, the method comprising:
acquiring information representing a user's exercise type; and
determining whether to calculate a movement amount of the wearable device per set intervals based on measurement results of the measurement unit during a period in which the positioning based on radio waves received from satellites is not obtained, in accordance with the user's exercise type,
**characterized in that**
when determining that the positioning based on radio waves received from satellites is intermittently performed with an idle period in between and the movement amount is not to be calculated based on measurement results of the measurement unit, performing additional positioning based on radio waves received from satellites if a change in traveling direction is detected by the measurement unit during the idle period.

7. A program executable by a computer in a wearable device that includes a positioning processing unit that performs positioning of the wearable device based on radio waves received from satellites and a measurement unit that performs measurements relating to an orientation and movement of the wearable device, the non-transitory storage medium storing a program executable by the computer, the program causing the computer to function as:
an acquisition unit that acquires information representing a user's exercise type; and
a calculation determination unit that determines whether to calculate a movement amount of the wearable device per set intervals based on measurement results of the physical sensor during a period in which the positioning based on radio waves received from satellites is not obtained, in accordance with the user's exercise type,
**characterized in that**
when determining that the positioning based on radio waves received from satellites is intermittently performed with an idle period in between and the movement amount is not to be calculated based on measurement results of the measurement unit, performing additional positioning based on radio waves received from satellites if a change in traveling direction is detected by the measurement unit during the idle period.

## Patentansprüche

1. Tragbare Vorrichtung, umfassend:
eine Positionierungsverarbeitungseinheit (18), die eine Positionierung der tragbare Vorrichtung auf der Grundlage von Funkwellen, die von Satelliten empfangen werden, durchführt;
eine Messeinheit (19), die Messungen in Bezug auf eine Ausrichtung und Bewegung der tragbare Vorrichtung durchführt;
eine Erfassungseinheit, die Informationen erfasst, die die Trainingsart eines Benutzers darstellen; und
eine Steuereinheit (11),
wobei die Steuereinheit bestimmt, ob eine Bewegungsmenge der tragbare Vorrichtung pro festgelegten Intervallen auf der Grundlage von Messergebnissen der Messeinheit während eines Zeitraums berechnet wird, in dem die Positionierung auf der Grundlage von Funkwellen, die von Satelliten empfangen werden, nicht erhalten wird, in Übereinstimmung mit der von der Erfassungseinheit erfassten Trainingsart des Benutzers,
**dadurch gekennzeichnet, dass**
wenn die Steuereinheit bestimmt, dass die Positionierung auf der Grundlage von Funkwellen, die von Satelliten empfang werden, mit einer dazwischen liegenden Ruhezeit intermittierend durchgeführt wird und die Bewegungsmenge nicht auf der Grundlage von Messergebnissen der Messeinheit berechnet werden soll, die Steuereinheit eine zusätzliche Positionierung auf der Grundlage von Funkwellen, die von Satelliten empfangen werden, durchführt, wenn während der Ruhezeit von der Messeinheit eine Änderung der Bewegungsrichtung detektiert wird.

2. Tragbare Vorrichtung nach Anspruch 1, wobei die Steuereinheit die Bewegungsmenge nicht auf der Grundlage von Messergebnissen der Messeinheit berechnet, wenn die Trainingsart des Benutzers eine Aktivität ist, bei der der Benutzer nicht läuft oder geht.

3. Tragbare Vorrichtung nach Anspruch 2, wobei die Steuereinheit, wenn die Bewegungsmenge nicht berechnet wird, eine Bewegungsdistanz zwischen zwei zeitlich benachbarten Punkten auf der Grundlage von Positionen der beiden Punkte berechnet, die durch Durchführung einer Positionierung auf der Grundlage von Funkwellen, die von Satelliten empfangen werden, erhalten werden.

4. Tragbare Vorrichtung nach Anspruch 1, wobei die Erfassungseinheit eine Betriebsempfangseinheit (15) enthält, die einen Eingabebetrieb empfängt, der die Trainingsart des Benutzers spezifiziert.

5. Tragbare Vorrichtung nach Anspruch 1, wobei der Messeinheitssensor mindestens einen von einem Beschleunigungssensor und einem Ausrichtungssensor enthält.

6. Verfahren zum Steuern einer Erfassung von Bewegungsinformationen einer tragbare Vorrichtung, das von der tragbare Vorrichtung durchgeführt wird, wobei die tragbare Vorrichtung eine Positionsverarbeitungseinheit (18), die eine Positionierung der tragbare Vorrichtung auf der Grundlage von Funkwellen, die von Satelliten empfangen werden, durchführt, und eine Messeinheit (19) enthält, die Messungen in Bezug auf eine Ausrichtung und Bewegung der tragbare Vorrichtung durchführt, wobei das Verfahren umfasst:
Erfassen von Informationen, die die Trainingsart eines Benutzers darstellen; und
Bestimmen, ob eine Bewegungsmenge der tragbare Vorrichtung pro festgelegten Intervallen auf der Grundlage von Messergebnissen der Messeinheit während eines Zeitraums berechnet werden soll, in dem die Positionierung auf der Grundlage von Funkwellen, die von Satelliten empfangen werden, nicht erhalten wird, in Übereinstimmung mit der Trainingsart des Benutzers,
**dadurch gekennzeichnet, dass**
wenn bestimmt wird, dass die Positionierung auf der Grundlage von Funkwellen, die von Satelliten empfangen werden, mit einer dazwischen liegenden Ruhezeit intermittierend durchgeführt wird und die Bewegungsmenge nicht auf der Grundlage von Messergebnissen der Messeinheit berechnet werden soll, eine zusätzliche Positionierung auf der Grundlage von Funkwellen, die von Satelliten empfangen werden, durchgeführt wird, wenn während der Ruhezeit von der Messeinheit eine Änderung der Bewegungsrichtung detektiert wird.

7. Programm, das von einem Computer in einer tragbare Vorrichtung ausgeführt werden kann, die eine Positionierungsverarbeitungseinheit, die eine Positionierung der tragbare Vorrichtung auf der Grundlage von Funkwellen, die von Satelliten empfangen werden, durchführt, und eine Messeinheit, die Messungen in Bezug auf eine Ausrichtung und Bewegung der tragbare Vorrichtung durchführt, enthält, wobei das nicht-transitorische Speichermedium ein Programm speichert, das von dem Computer ausgeführt werden kann, wobei das Programm den Computer dazu veranlasst, zu funktionieren als:
eine Erfassungseinheit, die Informationen erfasst, die die Trainingsart eines Benutzers darstellen; und
eine Berechnungsbestimmungseinheit, die bestimmt, ob eine Bewegungsmenge der tragbare Vorrichtung pro festgelegten Intervallen auf der Grundlage der Messergebnisse des physikalischen Sensors während eines Zeitraums berechnet werden soll, in dem die Positionierung auf der Grundlage von Funkwellen, die von Satelliten empfangen werden, nicht erhalten wird, in Übereinstimmung mit der Trainingsart des Benutzers,
**dadurch gekennzeichnet, dass**
wenn bestimmt wird, dass die Positionierung auf der Grundlage von Funkwellen, die von Satelliten empfangen werden, mit einer dazwischen liegenden Ruhezeit intermittierend durchgeführt wird und die Bewegungsmenge nicht auf der Grundlage von Messergebnissen der Messeinheit berechnet werden soll, eine zusätzliche Positionierung auf der Grundlage von Funkwellen, die von Satelliten empfangen werden, durchgeführt wird, wenn während der Ruhezeit von der Messeinheit eine Änderung der Bewegungsrichtung detektiert wird.

## Revendications

1. Dispositif portable, comprenant :
une unité d'exécution de positionnement (18) qui effectue un positionnement du dispositif portable sur la base d'ondes radio reçues depuis des satellites ;
une unité de mesure (19) qui effectue des mesures relatives à une orientation et un mouvement du dispositif portable ;
une unité d'acquisition qui acquiert des informations représentant un type d'exercice d'un utilisateur ; et
une unité de commande (11),
l'unité de commande déterminant s'il faut calculer une quantité de mouvement du dispositif portable par intervalles fixés, sur la base de résultats de mesure de l'unité de mesure pendant une période au cours de laquelle le positionnement basé sur des ondes radio reçues depuis des satellites n'est pas obtenu, en fonction du type d'exercice de l'utilisateur, acquis par l'unité d'acquisition,
**caractérisé en ce que**
lorsque l'unité de commande détermine que le positionnement basé sur des ondes radio reçues depuis des satellites est effectué par intermittence avec une période d'inactivité intercalée et la quantité de mouvement ne doit pas être calculée sur la base de résultats de mesure de l'unité de mesure, l'unité de commande effectue un positionnement supplémentaire basé sur des ondes radio reçues depuis des satellites si un changement de direction de déplacement est détecté par l'unité de mesure pendant la période d'inactivité.

2. Dispositif portable selon la revendication 1, dans lequel l'unité de commande ne calcule pas la quantité de mouvement sur la base de résultats de mesure de l'unité de mesure lorsque le type d'exercice de l'utilisateur est une activité ne faisant pas intervenir la marche ou la course de l'utilisateur.

3. Dispositif portable selon la revendication 2, dans lequel lorsque la quantité de mouvement n'est pas calculée, l'unité de commande calcule une distance de mouvement entre deux points adjacents dans le temps sur la base de positions des deux points obtenus en effectuant un positionnement basé sur des ondes radio reçues depuis des satellites.

4. Dispositif portable selon la revendication 1, dans lequel l'unité d'acquisition comprend une unité de réception (15) d'opérations qui reçoit une opération d'entrée spécifiant le type d'exercice de l'utilisateur.

5. Dispositif portable selon la revendication 1, dans lequel le capteur d'unité de mesure comprend au moins un parmi un capteur d'accélération et un capteur d'orientation.

6. Procédé de commande d'acquisition d'informations de mouvement d'un dispositif portable, exécuté par le dispositif portable, le dispositif portable comprenant une unité d'exécution de positionnement (18) qui exécute un positionnement du dispositif portable sur la base d'ondes radio reçues depuis des satellites et une unité de mesure (19) qui effectue des mesures relatives à une orientation et un mouvement du dispositif portable, le procédé comprenant les étapes consistant à :
acquérir des informations représentant un type d'exercice d'un utilisateur ; et
déterminer s'il faut calculer une quantité de mouvement du dispositif portable par intervalles définis, sur la base de résultats de mesure de l'unité de mesure pendant une période au cours de laquelle le positionnement basé sur des ondes radio reçues depuis des satellites n'est pas obtenu, en fonction du type d'exercice de l'utilisateur,
**caractérisé en ce que**
lorsqu'il est déterminé que le positionnement basé sur des ondes radio reçues depuis des satellites est effectué de façon intermittente avec une période d'inactivité intercalée et la quantité de mouvement ne doit pas être calculée sur la base de résultats de mesure de l'unité de mesure, effectuer un positionnement supplémentaire basé sur des ondes radio reçues depuis des satellites si un changement de direction de déplacement est détecté par l'unité de mesure au cours de la période d'inactivité.

7. Programme exécutable par un ordinateur dans un dispositif portable qui comprend une unité d'exécution de positionnement qui exécute le positionnement du dispositif portable sur la base d'ondes radio reçues depuis des satellites et une unité de mesure qui effectue des mesures relatives à une orientation et à un mouvement du dispositif portable, le support d'informations non transitoire mémorisant un programme exécutable par l'ordinateur, le programme amenant l'ordinateur à fonctionner comme :
une unité d'acquisition qui acquiert des informations représentant un type d'exercice d'un utilisateur ; et
une unité de détermination de calcul qui détermine s'il faut calculer une quantité de mouvement du dispositif portable par intervalles définis, sur la base de résultats de mesure du capteur physique pendant une période au cours de laquelle le positionnement basé sur des ondes radio reçues depuis des satellites n'est pas obtenu, en fonction du type d'exercice de l'utilisateur,
**caractérisé en ce que**
lorsqu'il est déterminé que le positionnement basé sur des ondes radio reçues depuis des satellites est effectué de façon intermittente avec une période d'inactivité intercalée et la quantité de mouvement ne doit pas être calculée sur la base de résultats de mesure de l'unité de mesure, effectuer un positionnement supplémentaire basé sur des ondes radio reçues depuis des satellites si un changement de direction de déplacement est détecté par l'unité de mesure au cours de la période d'inactivité.
